# EUROPÄISCHE PATENTSCHRIFT

(11) **EP 2 545 019 B1**
(45) Veröffentlichungstag und Bekanntmachung des Hinweises auf die Patenterteilung: **05.03.2014**
(21) Anmeldenummer: 11709361.7
(22) Anmeldetag: 08.03.2011
(51) Int. Cl.: C07C 7/08, C07C 11/167, B01D 3/40

(54) **VERFAHREN ZUR DESTILLATIVEN GEWINNUNG VON REIN-1,3-BUTADIEN AUS ROH-1,3-BUTADIEN**
METHOD FOR DISTILLATIVE EXTRACTION OF PURE 1,3-BUTADIENE FROM RAW 1,3-BUTADIENE
PROCEDE DE PRODUCTION PAR DISTILLATION DE 1,3-BUTADIENE PUR A PARTIR DE 1,3-BUTADIENE BRUT

(30) Priorität: 11.03.2010 DE 102010011014
(43) Veröffentlichungstag der Anmeldung: 16.01.2013
(73) Patentinhaber: BASF SE, 67056 Ludwigshafen (DE)
(72) Erfinder: HEIDA, Bernd, 67158 Ellerstadt (DE); HUGO, Randolf, 67246 Dirmstein (DE)
(86) Internationale Anmeldenummer: PCT/EP2011/053473
(87) Internationale Veröffentlichungsnummer: WO 2011/110562

(56) Entgegenhaltungen:
- WO-A1-2010/008109
- FR-A1- 2 440 984
- US-A- 4 277 313
- US-A1- 2005 240 071
- US-B1- 6 337 429
- US-B2- 7 226 527

## Beschreibung

Die Erfindung betrifft ein Verfahren zur Gewinnung von Rein-1,3-Butadien durch Extraktivdestillation mit einem selektiven Lösungsmittel aus einem C₄-Schnitt.

1,3-Butadien wird großtechnisch in der Regel aus so genannten C₄-Schnitten gewonnen, d. h. aus Gemischen von Kohlenwasserstoffen, worin die C₄-Kohlenwasserstoffe, insbesondere 1-Buten, i-Buten sowie 1,3-Butadien überwiegen. Neben geringen Mengen an C₃- und C₅-Kohlenwasserstoffen enthalten C₄-Schnitte in der Regel Acetylene, darunter Propin und Butine, insbesondere 1-Butin (Ethylacetylen) und Butenin (Vinylacetylen). Hierbei fällt zunächst ein sogenanntes 1,3-Roh-Butadien an, d. h. ein Gemisch, das etwa 89 bis 99,5 Gew.% 1,3-Butadien, Rest Verunreinigungen, enthält. Dieses muss anschließend, um den Spezifikationsanforderungen zu entsprechen, zu so genanntem 1,3-Rein-Butadien weiter destillativ aufgereinigt werden. Die Spezifikationsanforderungen für 1,3-Rein-Butadien sehen häufig einen Mindestgehalt an 1,3- Butadien von 99,6 Gew.-%, und einen maximal zulässigen Gehalt an Acetylenen sowie an 1,2-Butadien von jeweils 20 ppm vor.

Die Gewinnung von 1,3-Roh-Butadien aus C₄-Schnitten ist wegen der geringen Unterschiede in den relativen Flüchtigkeiten der Komponenten ein kompliziertes destillationstechnisches Problem und wird daher in der Regel durch sogenannte Extraktivdestillation mit einem selektiven Lösungsmittel durchgeführt.

Da der Gehalt an Acetylenen in C₄-Schnitten, darunter insbesondere Propin, 1-Butin und Butenin C₄-Schnitt in der Regel zwischen 0,5 und 3 Gew.-%, bezogen auf das Gesamtgewicht des C₄-Schnittes, beträgt, müssen die Acetylene, um die Spezifikationsanforderungen für Rein-1,3-Butadien zu erfüllen, aus dem C₄-Schnitt abgetrennt werden. Diese Abtrennung der Acetylene kann durch eine der Extraktivdestillation vorgeschaltete, so genannte Front-end-Selektivhydrierung durchgeführt werden, die den Vorteil hat, dass der apparative Aufwand sowie das Sicherheitsrisiko in der nachgeschalteten Extraktivdestillation signifikant erniedrigt und die Ausbeute an Raffinat 1, umfassend im Wesentlichen die Butane und die Butene sowie an 1,3-Butadien erhöht wird. Darüber hinaus werden bei der Front-end-Selektivhydrierung die acetylenischen C₄-Verunreinigungen, 1-Butin und Butenin in Wertprodukte übergeführt.

Die Front-end-Selektivhydrierung hat jedoch den Nachteil, dass der Anteil an Acetylenen im Feedstrom zur Selektivhydrierung, d.h. im C₄-Schnitt, niedrig ist, so dass für die Front-end-Selektivhydrierung hochselektive Katalysatoren und aufwändige Apparate erforderlich sind.

Ein Verfahren zur Frontend-Selektivhydrierung ist beispielsweise in US 4,277,311 beschrieben oder auch in der CN 101 172 929, wonach in einem ersten Verfahrensschritt der Anteil an Acetylenen durch Selektivhydrierung in C₄-Schnitt auf 5 bis 50 ppm reduziert wird, bei einem Butadienverlust von unter 1,5 Gew.-%.

Es war demgegenüber Aufgabe der Erfindung, ein verbessertes Verfahren zur Gewinnung von Rein-1,3-Butadien aus einem C₄-Schnitt mit Front-end-Selektivhydrierung der Acetylene zur Verfügung zu stellen, das gegenüber bekannten Verfahren mit weniger hochselektiven Katalysatoren, sowie in apparativ einfacherweise, durchführbar ist.

Diese Aufgabe wird gelöst durch ein Verfahren zur Gewinnung von Rein-1,3-Butadien durch Extaktivdestillation mit einem selektiven Lösungsmittel aus einem C₄-Schnitt, der neben 1,3-Butadien Butane, Butene und 0,5 bis 3 Gew.% Acetylene, bezogen auf das Gesamtgewicht des C₄-Schnittes, enthält, das dadurch gekennzeichnet ist, dass
- in einem ersten Verfahrensschritt ein Anteil von 90 bis 99 Gew.-%, bezogen auf das Gesamtgewicht der im C₄-Schnitt enthaltenen Acetylene selektiv hydriert wird, unter Erhalt eines partiell hydrierten C₄-Schnittes, der
- anschließend in weiteren Verfahrensschritten durch Extraktivdestillation mit dem selektiven Lösungsmittel und
- Reindestillation zu Rein-1,3-Butadien aufgearbeitet wird, wobei die im partiell hydrierten C₄-Schnitt verbliebenen Acetylene in den Verfahrensschritten, die sich an die partielle Hydrierung anschließen, ausgeschleust werden.

Es wurde gefunden, dass es möglich ist eine lediglich partielle Front-end-Selektivhydrierung durchzuführen, wobei die nach der partiellen Selektivhydrierung verbliebenen Acetylene in Apparaten ausgeschleust werden können, die für die nachfolgenden Verfahrensschritte bereits für andere Tennaufgaben vorhanden waren.

Der Anteil an Acetylenen im C₄-Schnitt liegt insbesondere im Bereich von 0,5 bis 1,5 Gew.-%, bezogen auf das Gesamtgewicht des C₄-Schnittes. Die Acetylene sind Propin sowie die C₄-Acetylene (Butine), d.h. 1-Butin (Ethylacetylen) und Butenin (Vinylacetylen).

Die partielle Front-end-Selektivhydrierung des C₄-Schnittes wird in der Regel in einem Reaktor durchgeführt, durch den der C₄-Schnitt und Wasserstoff über einen geeigneten heterogenen Katalysator geleitet werden. Hierzu eignet sich insbesondere auf Aluminiumoxid geträgertes Palladium.

Aus dem partiell hydrierten C₄-Schnitt wird anschließend Roh-1,3-Butadien durch Extraktivdestillation mit einem selektiven Lösungsmittel gewonnen, das anschließend zu Rein-1,3-Butadien destillativ aufgereinigt wird.

Für die Extraktivdestillation kann grundsätzlich jedes bekannte Verfahren eingesetzt werden.

Den Verfahren zur Extraktivdestillation von C₄-Schnitten unter Verwendung von selektiven Lösungsmitteln ist gemeinsam, dass sich durch Gegenstromführung des aufzutrennenden C4-Schnittes in Dampfform mit dem flüssigen selektiven Lösungsmittel bei geeigneten thermodynamischen Bedingungen, in der Regel bei niedrigen Temperaturen, meist im Bereich von 20 bis 80°C und bei moderaten Drücken, häufig bei etwa 3 bis etwa 6 bar, das selektive Lösungsmittel mit den Komponenten aus dem C4-Schnitt belädt, zu denen es eine höhere Affinität hat, wogegen die Komponenten, zu denen das selektive Lösungsmittel eine geringere Affinität hat, in der Dampfphase verbleiben und als Kopfstrom abgezogen werden. Aus dem beladenen Lösungsmittelstrom werden anschließend unter geeigneten thermodynamischen Bedingungen, d.h. bei höherer Temperatur und/oder niedrigerem Druck gegenüber dem ersten Verfahrensschritt, in einem oder mehreren weiteren Verfahrensschritten die Komponenten fraktioniert aus dem selektiven Lösungsmittel freigesetzt.

Bevorzugt enthält das selektive Lösungsmittel für die Extraktivdestillation 85 bis 95 Gew.-% NMP und 6 bis 12 Gew.-% Wasser. Darüber hinaus kann das selektive Lösungsmittel insbesondere noch Hilfsstoffe, Antischaummittel, organische Nebenkomponenten als Verunreinigung enthalten.

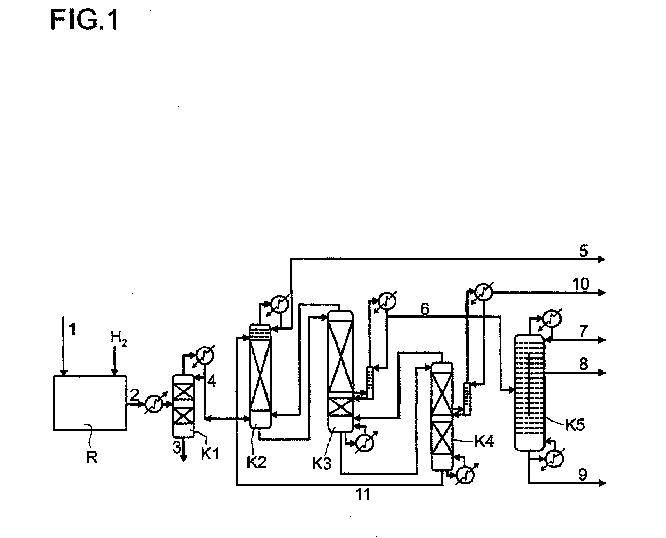

Die Extraktivdestillation von C₄-Schnitten wird häufig in der Weise gefahren, dass die Komponenten des C4-Schnittes, für die das selektive Lösungsmittel eine geringere Affinität als für 1,3-Butadien hat, insbesondere die Butane und die Butene, im Wesentlichen in der Gasphase verbleiben, wogegen 1,3-Butadien sowie Acetylene und weitere Kohlenwasserstoffe, für die das selektive Lösungsmittel eine höhere Affinität als für 1,3-Butadien hat, vom selektiven Lösungsmittel im Wesentlichen vollständig absorbiert werden. Die Gasphase wird als Kopfstrom abgezogen und häufig als Raffinat 1 bezeichnet. Ein derartiges Verfahren ist beispielsweise in der DE-A 198 188 10 beschrieben, wobei das Raffinat 1 der in den Figuren 1 und 2 mit Gbc bezeichnete Kopfstrom der Extraktivdestillationskolonne E I ist.

Die Aufarbeitung des mit 1,3-Butadien sowie mit weiteren Kohlenwasserstoffen, für die das selektive Lösungsmittel eine höhere Affinität als für 1,3-Butadien hat beladenen selektiven Lösungsmittels erfolgt in der Regel durch fraktionierte Desorption, wobei die im selektiven Lösungsmittel absorbierten Kohlenwasserstoffe in der umgekehrten Reihenfolge ihrer Affinität zum selektiven Lösungsmittel desorbiert werden.

Ein derartiges Verfahren ist beispielsweise in DE-A 198 188 10 beschrieben, wonach das selektive Lösungsmittel, das mit 1,3-Butadien und mit sonstigen C4-Kohlenwasserstoffen beladen ist, und als sogenannte Extraktionslösung ad bezeichnet ist, in einer Verfahrensstufe 3 in eine Desorptionszone mit gegenüber der Extraktionszone vermindertem Druck und/oder erhöhter Temperatur überführt und dabei aus der Extraktionslösung ad 1,3-Butadien desorbiert wird, wobei der Hauptteil der sonstigen C4-Kohlenwasserstoffe in der flüssigen Phase verbleibt. Dabei werden zwei getrennte Ströme abgezogen, und zwar 1,3-Butadien als Roh-1,3-Butadien-Strom und das mit sonstigen C4-Kohlenwasserstoffen beladene selektive Lösungsmittel als Extraktionslösung d. Aus der Extraktionslösung d werden schließlich in einer zweiten Desorptionszone mit gegenüber der ersten Desorptionszone vermindertem Druck und/oder erhöhter Temperatur und mit einem Druck- und/oder Temperaturgradienten noch darin verbliebenes 1,3-Butadien und die sonstigen C4-Kohlenwasserstoffe mindestens als zwei getrennte Fraktionen fraktioniert desorbiert.

Bevorzugt wird die Extraktivdestillation in einer Extraktivdestillationskolonne und im oberen Teil einer Extraktivdestillation- und Vorausgaserkolonne in der Weise durchgeführt, dass die Komponenten des C₄-Schnittes, für die das selektive Lösungsmittel eine geringere Affinität als für 1,3-Butadien hat, insbesondere die Butane und die Butene, im Wesentlichen in der Gasphase verbleiben und als Kopfstrom aus der Extraktivdestillationskolonne abgezogen werden, wogegen 1,3-Butadien, sowie die weiteren KohlenWasserstoffe, für die das selektive Lösungsmittel eine höhere Affinität als für 1,3-Butadien hat, vom selektiven Lösungsmittel im Wesentlichen vollständig absorbiert werden, wobei vom unteren Ende des oberen Bereichs der Extraktivdestillations- und Vorausgaserkolonne ein Roh-1,3-Butadienstrom abgezogen wird und im unteren Bereichder Extraktivdestillations- und Vorausgaserkolonne sowie in der Ausgaserkolonne die im selektiven Lösungsmittel absorbierten Kohlenwasserstoffe in der umgekehrten Reihenfolge ihre Affinität zum selektiven Lösungsmittel desorbiert werden, unter Erhalt eines Stromes von gereinigtem selektiven Lösungsmittel, der in die Extraktivdestillationskolonne recycliert wird, und wobei der Roh-1,3-Butadienstrom einer Reindestillationskolonne zugeführt wird, in der Rein-1,3-Butadien gewonnen wird.

Bei der obigen Verfahrensweise werden die im partiell hydrierten C₄-Schnitt verbliebenen C₄-Acetylene zum Teil über eine Seitenkolonne, die der Ausgaserkolonne zuge- ordnet ist, aus der das gereinigte selektive Lösungsmittel abgezogen wird und im übrigen über den Sumpfstrom der Reindestillationskolonne ausgeschleust.

Propin wird als Leichtsieder über den Kopfstrom der Reindestillationskolonne abgezogen.

In einer weiteren bevorzugten Verfahrensvariante wird die Extraktivdestillation des partiell hydrierten C₄-Schnittes zur Gewinnung von Roh-1,3-Butadiene in einer Trennwandkolonne durchgeführt, in der eine Trennwand in Kolonnenlängsrichtung unter Ausbildung eines ersten Teilbereichs, eines zweiten Teilbereichs und eines unteren gemeinsamen Kolonnenbereichs angeordnet und der eine Extraktivwaschkolonne vorgeschaltet ist, dadurch gekennzeichnet, dass man durch Regelung des Energieeintrags in die Trennwandkolonne über einen Sumpfverdampfer und Auslegung der Zahl der theoretischen Trennstufen im unteren gemeinsamen Kolonnenbereich die Fahrweise der Trennwandkolonne so einstellt, dass man aus der Trennwandkolonne einen Sumpfstrom erhält, der aus gereinigtem Lösungsmittel besteht. Diese Verfahrensvariante ist als so genannte kompressorlose Fahrweise bekannt und beispielsweise in EP-A 1 628 940 beschrieben.

Die Erfindung wird im Folgenden anhand einer Figur sowie eines Ausführungsbeispiels näher erläutert.

Die einzige Figur 1 zeigt die schematische Darstellung einer bevorzugten Anlage zur Durchführung des erfindungsgemäßen Verfahrens.

Ein C₄-Schnitt, Strom 1, wird, zusammen mit Wasserstoff, H₂, einem Reaktor R zur Durchführung einer partiellen Front-end-Selektivhydrierung zugeführt. Aus dem Reaktor R wird ein partiell hydrierter C₄-Schnitt abgezogen, der einer Schwersiederkolonne K1 zugeführt wird, worin die Schwersieder über Sumpf, als Strom 3 abgezogen werden und ein von Schwersiedern abgereicherter Strom 4 der Extraktivdestillation in einer Kolonne K2 zugeführt wird, aus der über Kopf so genanntes Raffinat 1, enthaltend die Butane und Butene wo, Strom 5, abgezogen wird.

In einer besonderen Ausführungsform besteht der Verstärkungsteil der Kolonne K1 nur aus einem Spritzschutz oder aus einem Demister und wird mit geringem Rücklauf betrieben. In einer weiteren besonderen Ausführungsform kann die Kolonne K1 ohne Verstärkungsteil gebaut und ohne Rücklauf gefahren werden.

Der Sumpfstrom aus der Extraktivdestillationskolonne K2 wird in der in der Figur dargestellten bevorzugten Ausführungsvariante der Extraktivdestillations- und Vorausgaserkolonne K3 zugeführt, aus der über einen Seitenabzug und eine kleine Kolonne mit Rückwaschböden, die das selektive Lösungsmittel zurückhalten, Roh-1,3-Butadien, Strom 6 entnommen wird, das einer Reindestillationskolonne K5, die in der in der Figur dargestellten bevorzugten Ausführungsform als Trennwandkolonne ausgebildet ist, in deren mittleren Bereich zugeführt wird. Der teilentgaste Sumpfstrom aus der Extraktivdestinations- und Vorausgaserkolonne K3 wird in den oberen Bereich der Ausgaserkolonne K4 aufgegeben, in der über Sumpf gereinigtes selektives Lösungsmittel abgezogen wird, das erneut in die Extraktivdestillationskolonne K2 recycliert wird.

Aus der Ausgaserkolonne K4 werden gegenüber 1,3-Butadien schwerflüchtige Komponenten, insbesondere C₅₊-Kohlenwasserstoffe, Carbonyle, etc. sowie C₄-Acetylene über einen Seitenabzug abgezogen, einer kleinen zur Rückwäsche von Lösungsmittel dienenden Seitenkolonne zugeführt, und über Kopf derselben als Strom 10 ausgeschleust.

In der Reindestillationskolonne K5, die bevorzugt als Trennwandkolonne ausgebildet ist, wird über den Kopfstrom 7 Propin und weitere gegenüber 1,3-Butadien leichter siedende Komponenten aus dem C₄-Schnitt ausgeschleust, über Sumpfstrom 9 Schwersieder und C₄-Acetylene, und über einen Seitenabzug aus dem Entnahmebereich der Trennwandkolonne spezifikationsgerechtes Rein-1,3-Butadien.

### Beispiel 1

Einer Anlage entsprechend der schematischen Darstellung in Figur 1 wurde ein C₄-Schnitt (Strom 1) mit den in der nachfolgenden Tabelle 1 aufgeführten Gewichtsanteilen in Gewichtsprozenten und Massenströmen in kg/h zugeführt:

Die Extraktivdestillationskolonne K2, die Extraktivdestillation- und Vorausgaserkolonne K3, sowie die Ausgaserkolonne K4, werden jeweils bei einem Kopfdruck von 4,8 bar absolut betrieben. Die Extraktivdestillationskolonne K2 umfasst 24 theoretische Trennstufen, die Extraktivdestillations- und Vorausgabekolonne K3 26 theorestische Trennstufen und die Ausgaserkolonne K4 14 theoretische Trennstufen.

Die Reindestillationskolonne K5 wird bei einem Kopfdruck von 5,3 bar absolut betrieben und umfasst 80 theoretische Trennstufen.

**Tabelle 1**

| | Gew.% | kg/h |
|---|---|---|
| Propan | 0,08 | 24,02 |
| Propen | 0,15 | 45,65 |
| Propadien | 0,07 | 21,01 |
| Propin | 0,22 | 65,19 |
| n-Butan | 7,04 | 2113,35 |
| iso-Butan | 2,63 | 789,50 |
| n-Buten | 12,90 | 3870,94 |
| iso-Buten | 25,87 | 7760,14 |
| trans-2-Buten | 4,81 | 1443,92 |
| cis-2-Buten | 3,57 | 1071,68 |
| 1,3-Butadien | 41,07 | 12322,17 |
| 1,2-Butadien | 0,21 | 63,04 |
| 1-Butin | 0,29 | 88,02 |
| Vinylacetylen | 0,94 | 282,45 |
| iso-Pentan | 0,05 | 15,01 |
| 3-Methylbuten-1 | 0,04 | 12,01 |
| 2-Methylbuten-2 | 0,04 | 12,01 |
| C8+ | | |
| H₂O | | |
| NMP | | |

Durch partielle Selektivhydrierung im Reaktor R wird ein Strom 2 gewonnen, der noch 18,01 kg/h Propin, 41 44 kg/h 1-Butine und 23,79 kg/h Vinylacetylen enthält. Der Umsatz an Vinylacetylen bei der Selektivhydrierung im Reaktor R beträgt 91,6 %.

Diese Restgehalte an Acetylenen werden in den nachfolgenden Verfahrensstufen nahezu vollständig ausgeschleust: Propin wird praktisch vollständig über den Kopfstrom 7 der Reinkolonne K5 abgetrennt, 1-Butin über die der Ausgaserkolonne K4 zugeordnete Seitenkolonne als Strom 10 (14,79 kg/h) sowie über den Sumpfstrom 9 der Reinkolonne K5 (26,59 kg/h).

Vinylacetylen (Butenin) wird über Strom 10 aus der der Ausgaserkolonne K4 zugeordneten Seitenkolonne (20,85 kg/h) und über den Sumpfstrom 9 der Reinkolonne K5 (2,85 kg/h) ausgeschleust.

Der Seitenabzug 8 aus der Reinkolonne K5 enthält spezifikationsgerechtes 1,3-Butadien, mit 99,72 Gew.-% 1,3-Butadien und daneben noch 0,28 Gew.-% Butene (Isobuten, trans-2-Buten und cis-2-Buten). Die Anteile an Acetylenen (Propin, 1-Butin und Vinylacetylen) liegen unterhalb der Grenzen für die geforderte Produktspezifikation.

### Beispiel 2

Der Anlage entsprechend der schematischen Darstellung in Figur 1 wird ein C4- Schnitt (Strom 1) mit den in der Tabelle 1 aufgeführten Zusammensetzung zugeführt: Der Umsatz an Vinylactylen bei der Selektivhydrierung im Reaktor R beträgt 95 %. Geringe Mengen an Vinylacetylen und Butin-1 werden über den Sumpf der Kolonne K1 zusammen mit höher siedenden Komponenten, z.B. C8-Komponenten, ausgeschleust. Somit beträgt der Zulaufstrom zur Extraktivdestillationskolonne K2 gemäß Figur 1 noch 14,9 kg/h Vinylacetylen, sowie 38,5 kg/h 1-Butin und 15,0 kg/h Propin.

Propin wird praktisch vollständig mit dem Kopfstrom 7 der Reinkolonne K5 abgezogen. 1-Butin wird über die der Ausgaserkolonne K4 zugeordnete Seitenkolonne als Strom 10 (9,3 kg/h) sowie über den Sumpfstrom 9 der Reinkolonne K5 (29,2 kg/h) abgezogen.

Vinylacetylen (Butenin) wird über Strom 10 aus der der Ausgaserkolonne K4 zugeordneten Seitenkolonne (13,4 kg/h) und über den Sumpfstrom 9 der Reinkolonne K5 (1,4 kg/h) ausgeschleust.

Der Seitenabzug 8 aus der Reinkolonne K5 enthält spezifikationsgerechtes 1,3-Butadien, mit mehr als 99,8 Gew.-% 1,3-Butadien. Zudem sind geringe Anteile an Butenen (n-Buten, Isobuten, trans-2-Buten und cis-2-Buten)enthalten. Die Anteile an Acetylenen (Propin, 1-Butin und Vinylacetylen) liegen jeweils unterhalb der Grenzen für die geforderte Produktspezifikation.

### Beispiel 3

Der Anlage gemäß Figur 1 wird ein C4-Schnitt (Strom 1) mit den in der Tabelle 1 aufgeführten Zusammensetzung zugeführt: Der Umsatz an Vinylactylen bei der Selektivhydrierung im Reaktor R beträgt 98 %. Der Zulaufstrom zur Extraktivdestillationskolonne K2 gemäß Figur 1 beträgt 5,7 kg/h Vinylacetylen, sowie 36,4 kg/h 1-Butin und 14,4 kg/h Propin.

Propin wird praktisch vollständig mit dem Kopfstrom 7 der Reinkolonne K5 abgezogen. 1-Butin wird über die der Ausgaserkolonne K4 zugeordnete Seitenkolonne als Strom 10 (20,6 kg/h) sowie über den Sumpfstrom 9 der Reinkolonne K5 (15,8 kg/h) abgezogen.

Vinylacetylen (Butenin) wird über Strom 10 aus der der Ausgaserkolonne K4 zugeordneten Seitenkolonne (5,2 kg/h) und über den Sumpfstrom 9 der Reinkolonne K5 (0,4 kg/h) ausgeschleust.

Der Seitenabzug 8 aus der Reinkolonne K5 enthält spezifikationsgerechtes 1,3-Butadien, mit mehr als 99,8 Gew.-% 1,3-Butadien. Zudem sind geringe Anteile an Butenen (n-Buten, Isobuten, trans-2-Buten und cis-2-Buten)enthalten. Die Anteile an Acetylenen (Propin, 1-Butin und Vinylacetylen) liegen jeweils unterhalb der Grenzen für die geforderte Produktspezifikation.

### Beispiel 4

Der Anlage gemäß Figur 1 wird ein C4-Schnitt (Strom 1) mit den in der Tabelle 1 aufgeführten Zusammensetzung zugeführt: Der Umsatz an Vinylactylen bei der Selektivhydrierung im Reaktor R beträgt 99 %. Der Zulaufstrom zur Extraktivdestillationskolonne K2 gemäß Figur 1 beträgt 2,7 kg/h Vinylacetylen, sowie 34,6 kg/h 1-Butin und 13,8 kg/h Propin.

Propin wird praktisch vollständig mit dem Kopfstrom 7 der Reinkolonne K5 abgezogen. 1-Butin wird über die der Ausgaserkolonne K4 zugeordnete Seitenkolonne als Strom 10 (16,1 kg/h) sowie über den Sumpfstrom 9 der Reinkolonne K5 (18,3 kg/h) abgezogen. Vinylacetylen (Butenin) wird über Strom 10 aus der der Ausgaserkolonne K4 zugeordneten Seitenkolonne (2, 5 kg/h) und über den Sumpfstrom 9 der Reinkolonne K5 (0,14 kg/h) ausgeschleust.

Der Seitenabzug 8 aus der Reinkolonne K5 enthält spezifikationsgerechtes 1,3-Butadien, mit mehr als 99,8 Gew.-% 1,3-Butadien. Zudem sind geringe Anteile an Butenen (n-Buten, Isobuten, trans-2-Buten und cis-2-Buten)enthalten. Die Anteile an Acetylenen (Propin, 1-Butin und Vinylacetylen) liegen jeweils unterhalb der Grenzen für die geforderte Produktspezifikation.

Die Beispiele 1 bis 4 zeigen somit, dass bei Umsätzen an Vinylacetylen über den gesamten beanspruchten Bereich, das heißt von 90 bis 99 %, bei der Reindestillation. stets spezifikationsgerechtes 1,3-Butadien erhalten wird.

## Patentansprüche

1. Verfahren zur Gewinnung von Rein-1,3-Butadien durch Extraktivdestillation mit einem selektiven Lösungsmittel aus einem C₄-Schnitt (1), der neben 1,3-Butadien Butane, Butene und 0,5 bis 3 Gew.% Acetylene, bezogen auf das Gesamtgewicht des C₄-Schnittes, enthält, wobei
- in einem ersten Verfahrensschritt ein Anteil von 90 bis 99 Gew.-%, bezogen auf das Gesamtgewicht der im C₄-Schnitt enthaltenen Acetylene selektiv hydriert wird, unter Erhalt eines partiell hydrierten C₄-Schnittes, der
- anschließend in weiteren Verfahrensschritten durch Extraktivdestillation mit dem selektiven Lösungsmittel und
- Reindestillation zu Rein-1,3-Butadien aufgearbeitet wird, wobei die im partiell hydrierten C₄-Schnitt verbliebenen Acetylene in den Verfahrensschritten, die sich an die partielle Hydrierung anschließen, ausgeschleust werden, wobei die Extraktivdestillation in einer Extraktivdestillationskolonne (K2) und im oberen Bereich einer Extraktivdestillations- und Vorausgaserkolonne (K3) in der Weise durchgeführt wird, dass die Komponenten des C₄-Schnittes (1), für die das selektive Lösungsmittel eine geringere Affinität als für 1,3-Butadien hat, insbesondere die Butane und die Butene, im Wesentlichen in der Gasphase verbleiben und als Kopfstrom (5) aus der Extraktivdestillationskolonne (K2) abgezogen werden, wogegen 1,3-Butadien, sowie die weiteren Kohlenwasserstoffe, für die das selektive Lösungsmittel eine höhere Affinität als für 1,3-Butadien hat, vom selektiven Lösungsmittel im Wesentlichen vollständig absorbiert werden, wobei
- vom unteren Ende des oberen Bereichs der Extraktivdestillations- und Vorausgaserkolonne (K3) ein Roh-1,3-Butadienstrom (6) abgezogen wird und im unteren Bereich der Extraktivdestillations- und Vorausgaserkolonne (K3) sowie in der Ausgaserkolonne (K4) die im selektiven Lösungsmittel absorbierten Kohlenwasserstoffe in der umgekehrten Reihenfolge ihre Affinität zum selektiven Lösungsmittel desorbiert werden, unter Erhalt eines Stromes von gereinigtem selektiven Lösungsmittel (11), der in die Extraktivdestillationskolonne (K2) recycliert wird, und wobei
- der Roh-1,3-Butadienstrom (6) einer Reindestillationskolonne (K5) zugeführt wird, in der Rein-1,3-Butadien (8) gewonnen wird, **dadurch gekennzeichnet, dass** die im partiell hydrierten C₄-Schnitt (2) verbliebenen C₄-Acetylene zum Teil über eine Seitenkolonne, die der Ausgaserkolonne (K4) zugeordnet ist, aus der das gereinigte selektive Lösungsmittel (11) abgezogen wird (Strom 10) und im übrigen über den Sumpfstrom (9) der Reindestillationskolonne (K5) ausgeschleust werden.

2. Verfahren nach Anspruch 1, **dadurch gekennzeichnet, dass** der C₄-Schnitt (1) einen Anteil von 0,5 bis 2,0 Gew.-% Acetylene, bezogen auf das Gesamtgewicht des C₄-Schnittes (1), enthält.

3. Verfahren nach einem der Ansprüche 1 oder 2, **dadurch gekennzeichnet, dass** Propin über den Kopfstrom (7) der Reindestillationskolonne (K5) abgezogen wird.

4. Verfahren nach einem der Ansprüche 1 bis 3, **dadurch gekennzeichnet, dass** das selektive Lösungsmittel 85 bis 95 Gew.-% NMP und 6 bis 12 Gew.-% Wasser enthält.

## Claims

1. A process for isolating pure 1,3-butadiene from a C₄ fraction (1) comprising 1,3-butadiene together with butanes, butenes and from 0.5 to 3% by weight of acetylenes, based on the total weight of the C₄ fraction, by extractive distillation using a selective solvent, wherein
- a proportion of from 90 to 99% by weight, based on the total weight of the acetylenes comprised in the C₄ fraction, is selectively hydrogenated in a first process step to give a partially hydrogenated C₄ fraction which
- is subsequently, in further process steps, worked up by extractive distillation using the selective solvent and
- pure distillation to give pure 1,3-butadiene, with the acetylenes remaining in the partially hydrogenated C₄ fraction being discharged in the process steps following the partial hydrogenation, where the extractive distillation is carried out in an extractive distillation column (K2) and in the upper region of an extractive distillation and preoutgassing column (K3) in such a way that the components of the C₄ fraction (1) for which the selective solvent has a lower affinity than for 1,3-butadiene, in particular the butanes and the butenes, remain essentially in the gas phase and are taken off as overhead stream (5) from the extractive distillation column (K2) while 1,3-butadiene and the further hydrocarbons for which the selective solvent has a higher affinity than for 1,3-butadiene are essentially completely absorbed by the selective solvent, where
- a crude 1,3-butadiene stream (6) is taken off at the lower end of the upper region of the extractive distillation and preoutgassing column (K3) and the hydrocarbons absorbed in the selective solvent are desorbed in the reverse order of their affinity for the selective solvent in the lower region of the extractive distillation and preoutgassing column (K3) and in the outgassing column (K4) to give a stream of purified selective solvent (11) which is recycled to the extractive distillation column (K2) and
- the crude 1,3-butadiene stream (6) is fed to a pure distillation column (K5) in which pure 1,3-butadiene (8) is isolated, wherein part of the C₄-acetylenes remaining in the partially hydrogenated C₄ fraction (2) is discharged via a side column assigned to the outgassing column (K4) from which the purified selective solvent (11) is taken off (stream 10) and the remainder is discharged via the bottom stream (9) from the pure distillation column (K5).

2. The process according to claim 1, wherein the C₄ fraction (1) comprises a proportion of from 0.5 to 2.0% by weight of acetylenes, based on the total weight of the C₄ fraction (1).

3. The process according to either of claims 1 and 2, wherein propyne is taken off via the overhead stream (7) from the pure distillation column (K5).

4. The process according to any of claims 1 to 3, wherein the selective solvent comprises from 85 to 95% by weight of NMP and from 6 to 12% by weight of water.

## Revendications

1. Procédé d'obtention de 1,3-butadiène pur par distillation extractive avec un solvant sélectif à partir d'une coupe en C₄ (1) qui contient, en plus du 1,3-butadiène, des butanes, des butènes et 0,5 à 3 % en poids d'acétylènes, par rapport au poids total de la coupe en C₄, selon lequel
- lors d'une première étape de procédé, une proportion de 90 à 99 % en poids, par rapport au poids total des acétylènes contenus dans la coupe en C₄, est hydrogénée sélectivement, pour obtenir une coupe en C₄ partiellement hydrogénée,
- qui est ensuite transformée lors d'étapes de procédé supplémentaires par distillation extractive avec le solvant sélectif et
- distillation pure en 1,3-butadiène pur, les acétylènes restants dans la coupe en C₄ partiellement hydrogénée étant évacués lors des étapes de procédé suivant l'hydrogénation partielle, la distillation extractive étant réalisée dans une colonne de distillation extractive (K2) et dans la zone supérieure d'une colonne de distillation extractive et de prédégazage (K3) de manière à ce que les composants de la coupe en C₄ (1), pour lesquels le solvant sélectif présente une affinité plus faible que pour le 1,3-butadiène, notamment les butanes et les butènes, restent essentiellement dans la phase gazeuse et soient soutirés de la colonne de distillation extractive (K2) sous la forme d'un courant de tête (5), tandis que le 1,3-butadiène, ainsi que les autres hydrocarbures, pour lesquels le solvant sélectif présente une affinité plus élevée que pour le 1,3-butadiène, sont absorbés essentiellement en totalité par le solvant sélectif,
- un courant de 1,3-butadiène brut (6) étant soutiré à l'extrémité inférieure de la zone supérieure de la colonne de distillation extractive et de pré-dégazage (K3), et les hydrocarbures absorbés dans le solvant sélectif étant désorbés dans l'ordre inverse de leur affinité pour le solvant sélectif dans la zone inférieure de la colonne de distillation extractive et de pré-dégazage (K3), ainsi que dans la colonne de dégazage (K4), pour obtenir un courant de solvant sélectif purifié (11), qui est recyclé dans la colonne de distillation extractive (K2), et
- le courant de 1,3-butadiène brut (6) étant introduit dans une colonne de distillation pure (K5), dans laquelle du 1,3-butadiène pur (8) est obtenu, **caractérisé en ce que** les acétylènes en C₄ restant dans la coupe en C₄ partiellement hydrogénée (2) sont évacués en partie par une colonne latérale, qui est attribuée à la colonne de dégazage (K4), à partir de laquelle le solvant sélectif purifié (11) est soutiré (courant 10), et pour le reste par le courant de fond (9) de la colonne de distillation pure (K5).

2. Procédé selon la revendication 1, **caractérisé en ce que** la coupe en C₄ (1) contient une proportion de 0,5 à 2,0 % en poids d'acétylènes, par rapport au poids total de la coupe en C₄ (1).

3. Procédé selon l'une quelconque des revendications 1 ou 2, **caractérisé en ce que** du propyne est soutiré par le courant de tête (7) de la colonne de distillation pure (K5).

4. Procédé selon l'une quelconque des revendications 1 à 3, **caractérisé en ce que** le solvant sélectif contient 85 à 95 % en poids de NMP et 6 à 12 % en poids d'eau.
